Europäisches Patentamt

European Patent Office

Office européen des brevets

(11) Publication number: **0 185 076 B1**

(12) **EUROPEAN PATENT SPECIFICATION**

(45) Date of publication of patent specification: **05.08.92**

(51) Int. Cl.5: **C12Q 1/02**, C07K 15/04, C12N 1/00

(21) Application number: **85903136.1**

(22) Date of filing: **07.06.85**

(86) International application number:
**PCT/US85/01081**

(87) International publication number:
**WO 86/00090 (03.01.86 86/01)**

(54) **HYBRID PROTEIN.**

(30) Priority: **07.06.84 US 618199**

(43) Date of publication of application:
**25.06.86 Bulletin 86/26**

(45) Publication of the grant of the patent:
**05.08.92 Bulletin 92/32**

(84) Designated Contracting States:
**AT BE CH DE FR GB IT LI LU NL SE**

(56) References cited:
**WO-A-83/03971**
**US-A- 4 468 382**

**ENDOCRINOLOGY, vol. 113, no. 3, 1983, pages 1072-1076, The Endocrine Society; P. BACHA et al.: "Organ-specific binding of a thyrotropin-releasing hormone-diphtheria toxin complex after intravenous administration to rats"**

(73) Proprietor: **SERAGEN, INC.**
**97 South Street**
**Hopkinton, MA 01748(US)**

(72) Inventor: **Murphy, John R., Dr.**
**15 Astra**
**Wayland, Massachusetts 01778(US)**

(74) Representative: **Sheard, Andrew Gregory et al**
**Kilburn & Strode 30, John Street**
**London WC1N 2DD(GB)**

Rank Xerox (UK) Business Services

N, Journal of Bacteriology, Volume 148, Pages 153-162, Issued October 1981, Gregory A. Buck et al, Identification of DNA Restriction Fragments of Beta-Converting Corynebacteriophages That Carry the Gene of Diphteria Toxin

N, Journal of Bacteriology, Volume 148, Pages 124-130, Issued October 1981, John J. Costa et al, Restriction Map of Coryne bacteriophages Beta-sub-C and Beta-sub-Vir and Physical Localization of the Diphteria Tox Operon.

N, Proceedings of the National Academy of Sciences, USA, Volume 79, Pages 2475-2479, Issued April 1982, MaSanory Suzuki et al, Production in Escherichia Coli of Biologically Active Secretin, A Gastrointestinal Hormone.

N, Journal of Cellular Biochemistry, Supplement, Volume 0, Number 6, page 133, Abstract 0364, Issued 1982, N. Shimuzu et al, Toxin-Hormone Conjugates and Resistant Variants.

N, Journal of Cellular Biochemistry, Volume 20, Pages 163-176, Issued 1982, Harvey R. Herschman et al, Toxic Ligand Conjugates as Tools in The Study of Receptor-Ligand Interactions.

N, Proceedings of the Miami Winter Symposia, Volume 19, Pages 275-288, Issued 1982, Diane M. Robins et al, Expression of the Human Growth Hormone Gene is Regulated in Mouse Cells.

N, The Harvey Lectures, Volume 76, Pages 45-74, Issued 1982, Alwin M. Pappenheimer, Diphteria: Studies on The Biology of an Infectious Disease.

N, Seminars in Infectious Disease, Volume 4, Pages 81-85, Issued 1982, John R. Murphy, Diphteria Toxin, Cross-Reacting Nontoxic Proteins, and The Development of New Biologicals. See page 85.

N, The Journal of Biological Chemistry, Volume 258, Pages 1565-1570, Issued 1983, Patricia Bacha et al, Thyrotropin-Releasing Hormone-Diphtria Toxin-Related Polypeptide Conjugates.

N, Endocrinology, Volume 113, Pages 1072-1076, Issued 1983, P. Bacha et al, Organ-Specific Binding of a Thyrotropin-Releasing Hormone-Diphteria Toxin Complex After Intravenous Administration to Rats.

N, Science, Volume 220, Pages 515-517, Issued 29 April 1983, Diane Leong et al, Cloned Fragment A of Diphtheria Toxin Is Expressed and Secreted Into the Periplasmic Space of Escherichia Coli K12.

N, Science, Volume 221, Pages 855-858, Issued 26 August 1983, Michel Kaczorek et al, Nucleotide Sequence and Expression of the Diphteria Tox 228 Gene in Escherichia Coli.

N, Applied and Environmental Microbiology, Volume 46, Pages 560-564, Issued September 1983, Rino Rappuoli, Isolation and Characterization of Corynebacterium Diphtheriae Nontandem Double Lysogens Hyperproducing CRM 197.

N, Infection and Immunity, Volume 42, Pages 48-56, Issued October 1983, Neal Groman et al, Detection and Expression of DNA-Homologous to the Tox Gene in Nontoxinogenic Isolates of Corynebacterium Diphteriae.

N, Journal of Bactriology, Volume 156, Pages 680-685, Issued November 1983, R. K. Tweten et al, Molecular Cloning and Expression of Gene Fragments From Corynebacteriophage Beta Encoding Enzymatically Active Peptides of Diphteria Toxin.

N, Proceedings of The National Academy of Sciences, USA, Volume 80, Pages 5863-6857, Issued November 1983, Lawrence Greenfield et al, Nucleotide Sequence of the Structural L Gene for Diphtheria Toxin carried by Coryne Bacteriophage Beta.

N, The Journal of Biological Chemistry, Volume 258, Pages 15016-15020, Issued 25 December 1983, Diane Leong et al, Cloned Diphtheria Toxin Fragment A is Expressed from the Tox Promoter and Exported to the Periplasm by the Sec-A Apparatus of Escherichia Coli K12. See page 10520.

**Description**

This invention relates to the use of recombinant DNA techniques to make hybrid protein molecules.

The literature contains many examples of fused genes which code for hybrid proteins. For example, Villa-Komaroff et al. (1978) P.N.A.S. U.S.A. 75, 3727-3731 describes a fused gene made up of a eukaryotic structural gene fused to a non-cytoplasmic bacterial gene. The fused gene codes for a hybrid protein which is transported out of the cytoplasm.

Hybrid proteins also have been made by other methods (e.g., the coupling of two different protein molecules) which do not involve recombinant DNA techniques. For example, it has been proposed to form, by coupling, therapeutic hybrid proteins consisting of portions of toxin molecules coupled to a ligand capable of binding specifically to a selected class of cells. One attempt to make such a hybrid protein, reported in Chang et al. (1977) J. Biol. chem 252, 1515-1522, resulted in a hybrid consisting of the diphtheria toxin A chain coupled to human placental lactogen hormone by cross-linking through a disulphide bond. The hybrid protein, although it bound to cells containing lactogen receptors, did not inhibit protein synthesis in those cells.

A hybrid protein consisting of the ricin toxin A chain coupled to the $\beta$ chain of human chorionic gonadotropin hormone by similarly cross-linking through a disulphide bond has also been reported; although said to have specifity, its binding capacity has not been reported. Furthermore, extremely high concentrations were required to significantly inhibit protein synthesis in rat Leydig tumour cells, making it difficult to distinguish between "non-specific" entry caused by endocytosis and "specific" entry caused by transport of the toxic portion of the hybrid across the cytoplasmic membrane of the target cells. Oeltman et al. (1979) J. Biol. Chem., 254, 1028-1032. The same shortcoming was found in a hybrid protein consisting of diphtheria A coupled to insulin using cystamine as the cross-linking agent (Miskimins et al. [1979] Biochem. Biophys. Res. Commun., 91, 143-151). A hybrid consisting of ricin A coupled to epidermal growth factor (EGF) by means of a heterobifunctional cross-linker has also been made but the binding characteristics provided by the EGF are not limited to specific cells, but rather encompass a wide variety of cell types (Cawley et al. [1980] Cell, 22, 563-570).

The present invention features, in general, a hybrid protein encoded by a fused gene; which fused gene comprises

a first sequence encoding a fragment of the diphtheria toxin molecule which renders the hybrid protein capable of penetrating the cytoplasmic membrane of a cell, fused to

a second sequence encoding a portion of a polypeptide ligand effective to enable the hybrid protein to bind selectively to the cell,

provided that the fused gene does not encode an enzymatically-active portion of diphtheria toxin Fragment A;

the hybrid protein optionally being detectably labelled. The hybrid protein can be labelled and used in diagnostic procedures, e.g., in vivo and in vitro imaging.

In preferred embodiments, the first sequence encodes less than all, and most preferably none, of the enzymatically active Fragment A of diphtheria toxin. Preferably the first sequence encodes a portion of Fragment B of diphtheria toxin sufficient to enable the hybrid protein to penetrate the cyotplasmic membrane of the cell; preferably this sequence encodes all of the amphipathic domain and at least a portion of the hydrophobic domain of Fragment B. The first sequence should not encode all of Fragment B, to ensure that binding of the hybrid protein is specifically directed by the polypeptide ligand and not by the generalized binding domain of diphtheria toxin.

The hybrid proteins of the invention can be labelled, e.g., with a radionucleotide, a dye, or a nuclear magnetic resonance (NMR) reporter group such as $^{13}C$. The hybrid protein binds selectively to the target cells for which the polypeptide ligand is specific, and then enters and becomes trapped in the cells, facilitating in vivo and in vitro imaging studies.

The invention also encompasses a method of labelling a class of cells by contacting the cells with a labelled hybrid protein as described broadly above, the polypeptide ligand of which has selective affinity for the class of cells. It will be understood that the polypeptide ligand may be a hormone.

The invention also relates to a cloning vector (for example a plasmid or a virus such as a phage) genetically incorporating a fused gene as broadly described.

The invention will be best understood by referring to the drawings and the following description, which are given by way of example.

In the drawings:

Fig. 1 is a diagrammatic representation of the diphtheria toxin molecule.

Fig. 2 is a restriction map showing the location and orientation of the diphtheria tox gene on the 3.9 kb BamH-I restriction fragment of corynephage $\beta^{tox}$.

Fig. 3 is the nucleotide sequence of the tox$^{228}$ allele and flanking regions, with amino acid residues shown above nucleotides: the B'' region is the region between the labeled Sau3AI and NruI sites (Fig. 3 was adapted from Fig. 1 of Kaczorek et al. [1983] Science 221, 855).

Fig. 4 is a diagrammatic representation of a plasmid containing the sequence encoding Fragment B.

Fig. 5 is a diagram of linkers which can be used to make fused genes of the invention.

Figs. 6 and 7 are diagrammatic representations of plasmids containing sequences encoding portions of Fragment B.

Fig. 8 is a diagrammatic represenation of a plasmid containing a sequence encoding alpha MSH.

Figs. 9 and 10 are diagrammatic representations of plasmids containing fused genes of the invention.

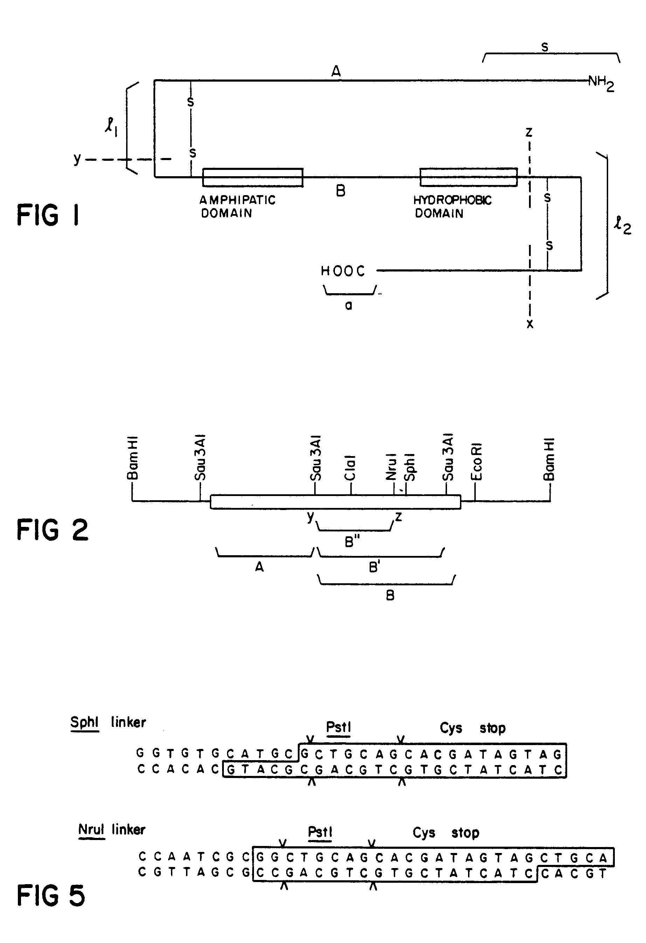

Referring to Fig. 1, the diphtheria toxin molecule consists of several functional "domains" which can be characterized, starting at the amino terminal end of the molecule, as hydrophobic leader signal sequence s, enzymatically active Fragment A, the fourteen amino acid exposed protease sensitive disulphide loop (DSL) l$_1$, containing a cleavage domain, and Fragment B, which includes the lipid associating regions, i.e., an amphipathic domain and a hydrophobic domain: DSL l$_2$; and carboxy terminal end a. DSL l$_1$ contains three arginine residues; the Sau3AI site between Fragment A and Fragment B (see Fig. 2) is at a position on the diphtheria toxin gene corresponding to the arginine residue farthest downstream of the three.

The process by which diphtheria toxin intoxicates sensitive eukaryotic cells involves at least the following steps: (i) diphtheria toxin binds to specific receptors on the surface of a sensitive cell; (ii) while bound to its receptor, the toxin molecule is internalized in an endocytic vesicle; (iii) either prior to internalization, or within the endocytic vesicle, the toxin molecule may be cleaved (or processed) at a site in the region of 47,000 daltons from the N-terminal end; (iv) as the pH of the endocytic vesicle decreases to below 6, the processed form of toxin, while still bound to its receptor, spontaneously inserts into the endosomal membrane; (v) once embedded in the membrane, the lipid associating regions form a pore; (vi) a proteolytic cleavage in l$_1$, between Fragment A and B, occurs; (vii) thereafter, Fragment A, or a polypeptide containing Fragment A, is released into the cytosol; (viii) the catalytic activity of Fragment A, i.e., the nicotinamide adenine dinucleotide - dependent adenosine diphosphate ribosylation of Elongation Factor 2. causes the death of the intoxicated cell. It is apparent that a single molecule of Fragment A introduced into the cytosol is sufficient to kill the cell.

Referring to Figs. 1 and 2, the hybrid protein of the invention preferably contains none of Fragment A of diphtheria toxin, and contains the portion of fragment B from y to z or from y to x (Fig. 1). The y-z portion is encoded by the Sau3A1-Nru1 sequence (Fig. 2) and the y-x portion, which includes l$_2$, is encoded by the Sau3A1-Sph1 sequence. It is most preferred that the hybrid protein contain y-z, to ensure that none of the generalized binding domain of Fragment B is included. In addition, any portion of Fragment B encoded by a sequence from Sau3A1 to a point no more than 180 base pairs upstream from Nru1 can be used. A portion shorter than this, e.g., that encoded by Sau3A1-Cla1, is too short to cause pore formation and therefore cannot be used. Portions of Fragment B encoded by a sequence ending downstream from Sph1 should not be used to avoid including the diphtheria toxin binding sites.

The part of the hybrid protein contributed by the polypeptide ligand can consist of the entire ligand, or a portion of the ligand which includes the entire binding domain of the ligand, or an effective portion of the binding domain.

When the ligand being used is large, it is desirable that as little of the non-binding portion as possible of the ligand be included, so that the binding domain of the molecule is positioned close to the hydrophobic domain of Fragment B. It is also desirable to include all or most of the binding domain of the ligand molecule. In the case of α melanocyte stimulating hormone (MSH), a small peptide of thirteen amino acids, or β MSH, which contains seventeen amino acids, the portion of the molecule consisting of nine amino acids at the carboxy terminal end of the molecule, which contains the receptor-specific binding domain, can be used, or the entire molecule can be used.

The regions within cell-specific ligands in which the binding domain is located are now known for a number of such ligands. Furthermore, recent advances in solid phase polypeptide synthesis can enable those skilled in this technology to determine the binding domain of practically any such ligand, by synthesizing various fragments of the ligand and testing them for the ability to bind to the class of cells to be labeled.

The detectable label which is attached to the hybrid protein molecule can be any conventional atom or molecule used for diagnostic labeling; examples are radioactive labels such as $^{125}$I-containing compounds, technitium isotopes, NMR reporter groups, and fluorescent dyes. The most preferred labels are hydrophobic labels such as fluorescent dyes (most conventional fluorescent dyes happen to be hydrophobic) which are incorporated into the cytoplasmic membrane of target cells, as will be explained in more detail below.

Labels can be attached to the hybrid protein according to conventional labeling techniques. Labels will be used in an amount (e.g. one or two label molecules per protein molecule) which does not interfere with the binding or cell penetration functions of the hybrid protein molecule.

The labeled hybrid protein of the invention can be used diagnostically to label a desired class of target cells. The specific binding domain of the polypeptide ligand portion selectively binds to those cells, and the labeled molecule is then taken up by the cells via endocytosis, and the label subsequently delivered to the cell membrane and/or the cytoplasm of the target cells.

The process by which a labelled hybrid protein of the invention can be incorporated into cells can be summarized as follows. The labelled hybrid protein is taken up by target cells via receptor-mediated endocytosis into an endocytic vesicle; thereafter a pH differential across the membrane of the endocytic vesicle is established as a result of the cell's ATP-dependent proton pump. The pH differential across the membrane causes the hybrid protein, including its lipid associating portion and its label, to be inserted into the plane of the membrane of the endocytic vesicle. The hydrophobic nature of the hybrid protein causes it to remain in the membrane, protected from the rapid enzymatic degradation which would occur were the labelled protein to reside in the cytoplasm or in the lumen of the endocytic vesicle.

After insertion into the plane of the membrane of the endocytic vesicle, the labelled hybrid protein can "traffic", as follows. The endocytic Vesicle, which buds off from the cytoplasmic membrane, enters the cytoplasm of the cell and can then merge with a lysosome, into which the labelled hybrid protein is incorporated. Alternatively, the endocytic vesicle can recycle to the cytoplasmic membrane of the cell. In either case, the label remains trapped in the target cell.

The hybrid proteins of the invention, in which fragments are joined via peptide bonds, are superior to cross-linked hybrids because the proteins of the invention can be provided in a homogeneous sample, in which all of the identical molecules are selective for a particular class of cells.

The specific class of cells which are bound and labelled by the hybrid proteins of the invention is determined by the specific polypeptide ligand which imparts the binding domain of the hybrid molecule. Any cell-specific polypeptide ligand can be used which has a binding domain which is specific for a particular class of cells which are to be labeled. Polypeptide hormones are useful such ligands. Hybrid proteins made using the binding domain of $\alpha$ or $\beta$ MSH, for example, can selectively bind to melanocytes, rendering the hybrids useful in the diagnosis of melanoma and the in vivo and in vitro detection of metastatic melanoma loci. Other ligands provide different specificities; e.g., the binding domain of substance P recognizes receptors on the surfaces of neurons involved in the transmission of pain, so that hybrids made using substance P can be used to map areas of the nervous system containing substance P receptors. Other specific-binding ligands which can be used include somatostatin, interleukin I, interleukin II, and interleukin III. Interleukin II is of particular importance because of its role in allergic reactions and autoimmune diseases such as Systemic Lupus Erythmatosis (SLE), involving activated T cells. Other useful polypeptide ligands having cell-specific binding domains are follicle stimulating hormone (specific for ovarian cells); luteinizing hormone (specific for ovarian cells); thyroid stimulating hormone (specific for thyroid cells); vasopressin (specific for uterine cells, as well as bladder and intestinal cells); prolactin (specific for breast cells); and growth hormone (specific for certain bone cells).

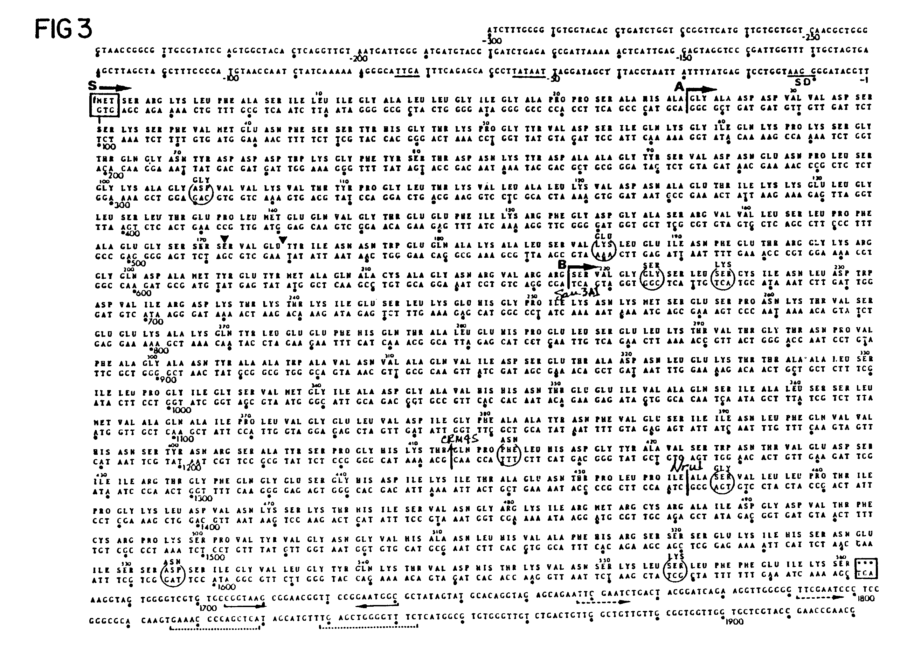

The hybrid proteins of the invention are made using recombinant DNA techniques involving forming the desired fused gene encoding the hybrid protein, and then expressing the fused gene, for example using conventional procedures. Referring to Figs. 2 and 3, the location and orientation of the diphtheria tox operon on the 3.9 kb BamH-I restriction fragment of corynephage $\beta^{tox+}$ allows the tox operon to be cleaved at a desired location, and the desired portion of the operon to be fused with the desired portion of the gene for a selected polypeptide ligand.

The preferred vectors for carrying out the necessary gene fusions are plasmids pUC7, pUC8, pUC9, and pBR322. The pUC plasmids, described in Viera et al. (1982) Gene 19, 259 (hereby incorporated by reference) are particularly well-suited for double digest cloning, a procedure which permits the fused genes to be made unambiguously.

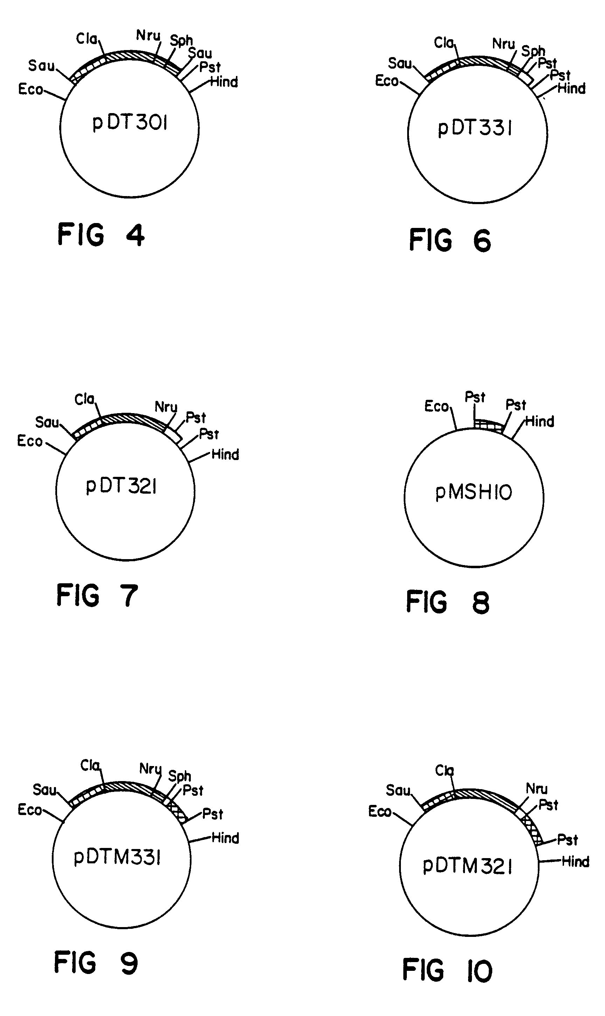

Gene fusions of the invention may be made as follows. First, a sequence encoding B' (Fig. 2), which includes all but the C-terminal 17 amino acids of fragment B. is cut out of the tox$^{228}$ allele and inserted in a plasmid This sequence (Sau3A1-Sau3A1, Fig. 2) carries the restriction endonuclease sites Cla1 and Sph1 (which are present on both the wild-type tox allele and the tox$^{228}$ allele), and the restriction endonuclease site Nru1 (which is unique to the tox$^{228}$ allele). The B' encoding sequence was cloned into the BamH1 site of pUC8 to yield pDT301 (Fig. 4). pDT301 was deposited in the American Type Culture Collection, Rockville, MD on May 11, 1983 and given ATCC Accession No. 39360.

The B' encoding sequence in pDT301 is then modified at the Nru1 or Sph1 restriction site to facilitate in-frame fusion with a gene sequence encoding the binding domain of a polypeptide ligand. When the Nru1 site is employed the resulting fusion encodes the B'' portion of Fragment B (from y to z in Fig. 1), which includes the amphipathic and hydrophobic domains of Fragment B, but not $I_2$. When the Sph1 site is used, the resulting fusion encodes the portion of Fragment B from y to x in Fig. 1; i.e., $I_2$ is included. The Cla1 site is not used because the resulting fusion would encode a portion of Fragment B lacking the hydrophobic domain, and thus would be incapable of causing pore formation in target cells.

Table 1 below summarizes the properties of the Fragment B-related proteins encoded by the above restriction fragments.

| fragment | no. amino acids encoded | amphipathic domain | hydrophobic domain | disulphide bridge $I_2$ |
|---|---|---|---|---|
| Sau3A1-1 (B') | 342 | + | + | + |
| Sau3A1-Sph1 | 291 | + | + | + |
| Sau3A1-Nru1 (B'') | 236 | + | + | - |
| Sau3A1-Cla1 | 96 | + | - | - |

Referring to Fig. 5, the B' encoding sequence is modified at either the Nru1 or the Sph1 restriction site by the introduction of a synthetic oligonucleotide which encodes a unique Pst1 restriction site followed by a Cys codon and a translational stop codon. This modification permits in-frame fusion with a gene sequence encoding the binding domain of a peptide ligand.

Figs. 6 and 7 illustrated plasmids made by carrying out the above modifications. Fig. 6 illustrates pDT33I, which contains the fragment B encoding sequence from Sau3AI to SphI and the SphI-PstI-Cys-Stop-Sma linker shown at the top of Fig. 5. Fig. 7 illustrates pDT32I, which contains the fragment B encoding sequence from Sau3AI to NruI and the NruI-Pst-Cys-Stop-Sma linker shown at the bottom of Fig. 5.

The PstI sites of pDT33I and pDT32I can serve as the sites for the in-frame insertion of a gene sequence encoding a peptide ligand binding domain, e.g., the sequence encoding melanocyte stimulating hormone (MSH). This sequence, with PstI restriction sites at each end, and a translational stop codon at the 5' end, is:

GCAAGTTATAGCATGGAACATTTTAGATGGGGAAAACCTGTATAGCTGCA

ACGTCGTTCAATATCGTACCTTGTAAAATCTACCCCTTTTGGACATATCG

This sequence, cloned in pUC8 to give pMSHI0, is illustrated in Fig. 8.

To make a fused gene of the invention, the MSH encoding sequence, above, can be cloned into the PstI site of pDT33I or pDT32I in the correct orientation. Insertion of the MSH sequence in pDT33I yields pDTM33I, illustrated in Fig. 9. Insertion of the MSH sequence in pDT32I yields pDTM32I, illustrated in Fig. 10. Both plasmids encode hybrid proteins which can selectively bind to and form pores in melanocytes.

pDTM33I has been deposited with the American Type Culture Collection on 12th June 1985 and given ATCC Accession Number ATC 53145.

Another example of a suitable polypeptide ligand is substance P (the utility of substance P is described above). A fused gene containing the substance P gene, rather than the $\alpha$ or $\beta$ MSH gene, is made basically as outlined above. The substance P gene can be synthesized using conventional solid phase oligonucleotide synthesis. The substance P gene sequence is: CGTCCTAAACCTCAGCAGTTCTTCGGTCTGATG.

As is clear from the above, the portion of the genetic sequence encoding the polypeptide ligand must be sufficient to enable the corresponding amino acid sequence to cause the hybrid protein to bind to the selected class of cells. Preferably, the gene includes all or most of the genetic sequence for the binding domain of the ligand.

Generally, as in the above examples, the manipulative operations are carried out using cloning vectors; e.g., phages or plasmids. The genetic material coding for the binding domain of the polypeptide ligand can be either cloned DNA or a synthetic oligonucleotide sequence. Generally the fused gene will reside on a cloning vector, e.g., a plasmid or a phage, which is used to transform cultured microorganisms or yeast or mammalian host cells. The hybrid protein is then harvested from the culture media of the cells using conventional techniques.

Purification of the hybrid proteins of the invention, regardless of the polypeptide ligand used, can be

EP 0 185 076 B1

carried out via affinity chromotography, using a monoclonal antibody against diphtheria toxin Fragment B.

As is mentioned above, a major diagnostic use of the labelled hybrid proteins will be the in vivo and in vitro detection of metastatic loci, using conventional cell staining and labelling techniques. Such detection could be of particular value in surgery, by providing the surgeon with information needed to know how much tissue to excise when removing. e.g., metastatic melanoma cells.

Other embodiments are within the following claims.

**Claims**
**Claims for the following Contracting States : BE, CH, DE, FR, GB, IT, LI, LU, NL, SE**

1. A hybrid protein encoded by a fused gene, which fused gene comprises

   a first sequence encoding a fragment of the diphtheria toxin molecule which renders the hybrid protein capable of penetrating the cytoplasmic membrane of a cell, fused to

   a second sequence encoding a portion of a polypeptide ligand effective to enable the hybrid protein to bind selectively to the cell,

   provided that the fused gene does not encode an enzymatically-active portion of diphtheria toxin Fragment A;

   the hybrid protein optionally being detectably labelled.

2. A hybrid protein as claimed in Claim 1 encoded by a fused gene wherein the first sequence encodes less than all, or none, of Fragment A of the diphtheria toxin molecule.

3. A hybrid protein as claimed in Claim 1 or Claim 2, encoded by a fused gene wherein the first sequence encodes a portion of Fragment B of diphtheria toxin sufficient to enable the hybrid protein to penetrate the cytoplasmic membrane of the cell and not encoding the generalized binding domain of Fragment B.

4. A hybrid protein as claimed in Claim 1 or Claim 3, encoded by a fused gene wherein the first sequence comprises the Sau3A1-Nru 1 sequence or the Sau3A1-Sph1 sequence of the $tox^{228}$ gene.

5. A hybrid protein as claimed in Claim 1 or Claim 3, encoded by a fused gene wherein the first sequence includes the sequence encoding the amphipathic domain and the hydrophobic domain of the diphtheria toxin molecule.

6. A hybrid protein as claimed in Claim 5, encoded by a fused gene wherein the first sequence further comprises a sequence encoding the disulphide loop $l_2$.

7. A hybrid protein as claimed in Claim 1, encoded by a fused gene wherein the first sequence encodes all of the amphipathic domain and at least a portion of the hydrophobic domain of Fragment B of diphtheria toxin, the first sequence including at least the portion of the Fragment B encoding region from the Sau3A1 site in the $l_1$-encoding region to a point not more than 180 base pairs upstream from the position corresponding to the Nru1 site on the $tox^{228}$ allele.

8. A hybrid protein as claimed in any one of Claims 1 to 7 wherein the hybrid protein is capable of being taken up by the cell by endocytosis.

9. A hybrid protein as claimed in Claim 7 or Claim 8 , wherein the label is a fluorescent dye.

10. A hybrid protein as claimed in Claim 9, wherein the dye, along with the hybrid protein, is capable of being inserted into the cytoplasmic membrane of the cell.

11. A hybrid protein as claimed in any preceding claim, wherein the polypeptide ligand is a hormone.

12. A hybrid protein as claimed in Claim 11, wherein the hormone is alpha or beta melanocyte stimulating hormone, interleukin I, interleukin II, interleukin III, or alpha or beta hCG.

13. A method of labelling a class of cells, comprising contacting the cells with a labelled hybrid protein as claimed in any preceding claim.

7

EP 0 185 076 B1

**14.** A method of making a hybrid protein as claimed in any one of claims 1 to 12, the method comprising expressing a fused gene.

**15.** A hybrid protein comprising a fragment of the diphtheria toxin molecule which renders the hybrid protein capable of penetrating the cytoplasmic membrane of a cell, providing that the fragment does not include any enzymatically active portion of diphtheria toxin Fragment A, or a segment of diphtheria toxin capable of binding to cells, the fragment being covalently linked to a portion of a polypeptide ligand effective to enable the hybrid protein to bind selectively to the cell.

**16.** A hybrid protein as claimed in claim 15, wherein the polypeptide ligand is alpha or beta melanocyte stimulating hormone, interleukin I, interleukin II, interleukin III or alpha or beta hCG.

**Claims for the following Contracting State : AT**

**1.** A process for the preparation of a hybrid protein encoded by a fused gene, which fused gene comprises
a first sequence encoding a fragment of the diphtheria toxin molecule which renders the hybrid protein capable of penetrating the cytoplasmic membrane of a cell, fused to
a second sequence encoding a portion of a polypeptide ligand effective to enable the hybrid protein to bind selectively to the cell,
provided that the fused gene does not encode an enzymatically-active portion of diphtheria toxin Fragment A;
the hybrid protein optionally being detectably labelled,
the process comprising expressing the fused gene.

**2.** A process as claimed in Claim 1 wherein the first sequence encodes less than all, or none, of Fragment A of the diphtheria toxin molecule.

**3.** A process as claimed in Claim 1 or 2, wherein the first sequence encodes a portion of Fragment B of diphtheria toxin sufficient to enable the hybrid protein to penetrate the cytoplasmic membrane of the cell and not encoding the generalized binding domain of Fragment B.

**4.** A process as claimed in Claim 1 or 3, wherein the first sequence comprises the Sau3A1-Nru 1 sequence or the Sau3A1-Sph1 sequence of the $tox^{228}$ gene.

**5.** A process as claimed in Claim 1 or Claim 3, wherein the first sequence includes the sequence encoding the amphipathic domain and the hydrophobic domain of the diphtheria toxin molecule.

**6.** A process as claimed in Claim 5, wherein the first sequence further comprises a sequence encoding the disulphide loop $l_2$.

**7.** A process as claimed in Claim 1, wherein the first sequence encodes all of the amphipathic domain and at least a portion of the hydrophobic domain of Fragment B of diphtheria toxin, the first sequence including at least the portion of the Fragment B encoding region from the Sau3A1 site in the $l_1$-encoding region to a point not more than 180 base pairs upstream from the position corresponding to the Nru1 site on the $tox^{228}$ allele.

**8.** A process as claimed in any one of Claims 1 to 7 wherein the hybrid protein is capable of being taken up by the cell by endocytosis.

**9.** A process as claimed in Claim 8, wherein the label is a fluorescent dye.

**10.** A process as claimed in Claim 9, wherein the dye, along with the hybrid protein, is capable of being inserted into the cytoplasmic membrane of the cell.

**11.** A process as claimed in any preceding claim wherein the polypeptide ligand is a hormone.

**12.** A process as claimed in Claim 11, wherein the hormone is alpha or beta melanocyte stimulating

8

hormone, interleukin I, interleukin II, interleukin III, or alpha or beta hCG.

13. A method of labelling a class of cells, comprising contacting the cells with a labelled hybrid protein preparable by a process as claimed in any one of Claims 1 to 12.

14. A process for the preparation of a hybrid protein comprising a fragment of the diphtheria toxin molecule which renders the hybrid protein capable of penetrating the cytoplasmic membrane of a cell, providing that the fragment does not include any enzymatically active portion of diphtheria toxin Fragment A, or a segment of diphtheria toxin capable of binding to cells, the fragment being covalently linked to a portion of a polypeptide ligand effective to enable the hybrid protein to bind selectively to the cell, the process comprising expressing DNA coding for the hybrid protein.

15. A process as claimed in claim 14, wherein the polypeptide ligand is alpha or beta melanocyte stimulating hormone, interleukin I, interleukin II, interleukin III or alpha or beta hCG.

**Revendications**
**Revendications pour les Etats contractants suivants : BE, CH, DE, FR, GB, IT, LI, LU, NL, SE**

1. Protéine hybride codée par un gène fusionné, lequel gène fusionné comprend :
   une première séquence codant un fragment de la molécule de la toxine diphtérique qui rend la protéine hybride capable de pénétrer à travers la membrane cytoplasmique d'une cellule, fusionnée avec :
   une deuxième séquence codant une partie d'un ligand polypeptidique efficace pour permettre à la protéine hybride de se lier sélectivement à la cellule,
   avec la restriction que le gène fusionné ne code pas une partie enzymatiquement active du fragment A de la toxine diphtérique,
   la protéine hybride étant facultativement marquée de façon détectable.

2. Protéine hybride suivant la revendication 1, codée par un gène fusionné dans lequel la première séquence code moins que la totalité sinon rien du fragment A de la molécule de la toxine diphtérique.

3. Protéine hybride suivant la revendication 1 ou 2, codée par un gène fusionné dans lequel la première séquence code une partie du fragment B de la toxine diphtérique suffisante pour permettre à la protéine hybride de pénétrer à travers la membrane cytoplasmique de la cellule et ne code pas le domaine de liaison généralisée du fragment B.

4. Protéine hybride suivant la revendication 1 ou 3, codée par un gène fusionné dans lequel la première séquence comprend la séquence Sau3A1-Nru1 ou la séquence Sau3A1-Sph1 du gène tox$^{228}$.

5. Protéine hybride suivant la revendication 1 ou 3, codée par un gène fusionné dans lequel la première séquence comprend la séquence codant le domaine amphipathique et le domaine hydrophobe de la molécule de la toxine diphtérique.

6. Protéine hybride suivant la revendication 5, codée par un gène fusionné dans lequel la première séquence comprend, en outre, une séquence codant la boucle disulfure $l_2$.

7. Protéine hybride suivant la revendication 1, codée par un gène fusionné dans lequel la première séquence code la totalité du domaine amphipathique et au moins une partie du domaine hydrophobe du fragment B de la toxine diphtérique, la première séquence comprenant au moins la partie de la région codant le fragment B depuis le site Sau3A1 dans la région codant $l_1$ jusqu'à un point en amont de 180 paires de bases au maximum de la position correspondant au site Nru1 sur l'allèle tox$^{228}$.

8. Protéine hybride suivant l'une quelconque des revendications 1 à 7, dans laquelle la protéine hybride est capable d'être absorbée par la cellule par endocytose.

9. Protéine hybride suivant la revendication 7 ou 8, dans laquelle le marqueur est un colorant fluorescent.

10. Protéine hybride suivant la revendication 9, dans laquelle le colorant, conjointement avec la protéine hybride, est capable d'être inséré dans la membrane cytoplasmique de la cellule.

11. Protéine hybride suivant l'une quelconque des revendications précédentes, dans laquelle le ligand polypeptidique est une hormone.

12. Protéine hybride suivant la revendication 11, dans laquelle l'hormone est l'hormone stimulatrice des mélanocytes $\alpha$ ou $\beta$, l'interleukine I, l'interleukine II, l'interleukine III ou l'$\alpha$ ou $\beta$ hCG.

13. Procédé pour marquer une classe de cellules, qui comprend la mise en contact des cellules avec une protéine hybride marquée suivant l'une quelconque des revendications précédentes.

14. Procédé pour former une protéine hybride suivant l'une quelconque des revendications 1 à 12, le procédé comprenant l'expression d'un gène fusionné.

15. Protéine hybride comprenant un fragment de la molécule de la toxine diphtérique qui rend la protéine hybride capable de pénétrer à travers la membrane cytoplasmique d'une cellule, avec la restriction que le fragment ne comprend aucune partie enzymatiquement active du fragment A de la toxine diphtérique, ni un segment de la toxine diphtérique capable de se lier aux cellules, le fragment étant uni par covalence à une partie d'un ligand polypeptidique efficace pour permettre à la protéine hybride de se lier sélectivement à la cellule.

16. Protéine hybride suivant la revendication 15, dans laquelle le ligand polypeptidique est l'hormone de stimulation des mélanocytes $\alpha$ ou $\beta$, l'interleukine I, l'interleukine II, l'interleukine III ou l'$\alpha$ ou $\beta$ hCG.

**Revendications pour l'Etat contractant suivant : AT**

1. Procédé de préparation d'une protéine hybride codée par un gène fusionné, lequel gène fusionné comprend :
   une première séquence codant un fragment de la molécule de la toxine diphtérique qui rend la protéine hybride capable de pénétrer à travers la membrane cytoplasmique d'une cellule, fusionnée avec :
   une deuxième séquence codant une partie d'un ligand polypeptidique efficace pour permettre à la protéine hybride de se lier sélectivement à la cellule,
   avec la restriction que le gène fusionné ne code pas une partie enzymatiquement active du fragment A de la toxine diphtérique,
   la protéine hybride étant facultativement marquée de façon détectable,
   le procédé comprenant l'expression du gène fusionné.

2. Procédé suivant la revendication 1, dans lequel la première séquence code moins que la totalité sinon rien du fragment A de la molécule de la toxine diphtérique.

3. Procédé suivant la revendication 1 ou 2, dans lequel la première séquence code une partie du fragment B de la toxine diphtérique suffisante pour permettre à la protéine hybride de pénétrer à travers la membrane cytoplasmique de la cellule et ne code pas le domaine de liaison généralisée du fragment B.

4. Procédé suivant la revendication 1 ou 3, dans lequel la première séquence comprend la séquence Sau3A1-Nru1 ou la séquence Sau3A1-Sph1 du gène tox$^{228}$.

5. Procédé suivant la revendication 1 ou 3, dans lequel la première séquence comprend la séquence codant le domaine amphipathique et le domaine hydrophobe de la molécule de la toxine diphtérique.

6. Procédé suivant la revendication 5, dans lequel la première séquence comprend, en outre, une séquence codant la bouche disulfure $l_2$.

7. Procédé suivant la revendication 1, dans lequel la première séquence code la totalité du domaine amphipathique et au moins une partie du domaine hydrophobe du fragment B de la toxine diphtérique, la première séquence comprenant au moins la partie de la région codant le fragment B depuis le site

10

Sau3A1 dans la région codant $I_1$ jusqu'à un point en amont de 180 paires de bases au maximum de la position correspondant au site Nru1 sur l'allèle tox$^{228}$.

**8.** Procédé suivant l'une quelconque des revendications 1 à 7, dans lequel la protéine hybride est capable d'être absorbée par la cellule par endocytose.

**9.** Procédé suivant la revendication 8, dans lequel le marqueur est un colorant fluorescent.

**10.** Procédé suivant la revendication 9, dans lequel le colorant, conjointement avec la protéine hybride, est capable d'être inséré dans la membrane cytoplasmique de la cellule.

**11.** Procédé suivant l'une quelconque des revendications précédentes, dans lequel le ligand polypeptidique est une hormone.

**12.** Procédé suivant la revendication 11, dans lequel l'hormone est l'hormone stimulatrice des mélanocytes $\alpha$ ou $\beta$, l'interleukine I, l'interleukine II, l'interleukine III ou l'$\alpha$ ou $\beta$ hCG.

**13.** Procédé pour marquer une classe de cellules, qui comprend la mise en contact des cellules avec une protéine hybride marquée qui peut être préparée suivant l'une quelconque des revendications 1 à 12.

**14.** Procédé de préparation d'une protéine hybride comprenant un fragment de la molécule de la toxine diphtérique qui rend la protéine hybride capable de pénétrer à travers la membrane cytoplasmique d'une cellule, avec la restriction que le fragment ne comprend aucune partie enzymatiquement active du fragment A de la toxine diphtérique, ni un segment de la toxine diphtérique capable de se lier aux cellules, le fragment étant uni par covalence à une partie d'un ligand polypeptidique efficace pour permettre à la protéine hybride de se lier sélectivement à la cellule, le procédé comprenant l'expression de l'ADN codant la protéine hybride.

**15.** Procédé suivant la revendication 14, dans lequel le ligand polypeptidique est l'hormone de stimulation des mélanocytes $\alpha$ ou $\beta$, l'interleukine I, l'interleukine II, l'interleukine III ou l'$\alpha$ ou $\beta$ hCG.

**Patentansprüche**
**Patentansprüche für folgende Vertragsstaaten : BE, CH, DE, FR, GB, IT, LI, LU, NL, SE**

**1.** Hybrides Protein, das durch ein Fusionsgen kodiert wird, wobei das Fusionsgen folgendes umfaßt:
eine erste Sequenz, die ein Fragment des Diphtherietoxinmoleküls kodiert, welches dem hybriden Protein die Fähigkeit verleiht, die zytoplasmatische Membran einer Zelle zu durchdringen, fusioniert mit einer zweiten Sequenz, die einen Teil eines Polypeptidliganden kodiert, der bewirkt, daß das hybride Protein selektiv an die Zelle binden kann,
unter der Voraussetzung, daß das Fusionsgen keinen enzymatisch aktiven Teil des Diphtherietoxin-Fragments A kodiert;
wobei das hybride Protein gegebenenfalls nachweisbar markiert ist.

**2.** Hybrides Protein nach Anspruch 1, das durch ein Fusionsgen kodiert wird, wobei die erste Sequenz nicht das gesamte oder nichts von Fragment A des Diphtherietoxinmoleküls kodiert.

**3.** Hybrides Protein nach Anspruch 1 oder Anspruch 2, das durch ein Fusionsgen kodiert wird, wobei die erste Sequenz einen Teil des Fragments B von Diphtherietoxin, der ausreicht, um dem hybriden Protein die Fähigkeit zu verleihen, die zytoplasmatische Membran der Zelle zu durchdringen, und nicht die allgemeine Bindungsregion von Fragment B kodiert.

**4.** Hybrides Protein nach Anspruch 1 oder Anspruch 3, das durch ein Fusionsgen kodiert wird, wobei die erste Sequenz die Sau3A1-Nru1-Sequenz oder die Sau3A1-Sph1-Sequenz des tox$^{228}$-Gens umfaßt.

**5.** Hybrides Protein nach Anspruch 1 oder Anspruch 3, das durch ein Fusionsgen kodiert wird, wobei die erste Sequenz jene Sequenz beinhaltet, welche die amphipatisehe Region und die hydrophobe Region des Diphtherietoxinmoleküls kodiert.

**6.** Hybrides Protein nach Anspruch 5, das durch ein Fusionsgen kodiert wird, wobei die erste Sequenz ferner eine Sequenz umfaßt, welche die DisulfidSchleife $I_2$ kodiert.

**7.** Hybrides Protein nach Anspruch 1, das durch ein Fusionsgen kodiert wird, wobei die erste Sequenz die gesamte amphipatische Region und zumindest einen Teil der hydrophoben Region von Fragment B von Diphtherietoxin kodiert, und wobei die erste Sequenz zumindest den Teil der kodierenden Region von Fragment B beinhaltet, der von der Sau3A1-Stelle in der $I_1$-kodierenden Region bis zu einem Punkt reicht, der nicht mehr als 180 Basenpaare von jener Position aufwärts liegt, die der Nru1-Stelle aufdem $tox^{228}$-Allel entspricht.

**8.** Hybrides Protein nach einem der Ansprüche 1 bis 7, wobei das hybride Protein von der Zelle durch Endocytose aufgenommen werden kann.

**9.** Hybrides Protein nach Anspruch 7 oder Anspruch 8, wobei der Marker ein Fluoreszenzfarbstoff ist.

**10.** Hybrides Protein nach Anspruch 9, wobei der Farbstoff gemeinsam mit dem hybriden Protein in die zytoplasmatische Membran der Zelle eingefügt werden kann.

**11.** Hybrides Protein nach einem der vorangehenden Ansprüche, wobei der Polypeptidligand ein Hormon ist.

**12.** Hybrides Protein nach Anspruch 11, wobei das Hormon Alpha- oder Beta-melanocytenstimulierendes Hormon, Interleukin I, Interleukin II, Interleukin III oder Alpha- oder Beta-HCG ist.

**13.** Methode zur Markierung einer Klasse von Zellen, wobei die Zellen mit einem markierten hybriden Protein nach einem der vorangehenden Ansprüche in Kontakt gebracht werden.

**14.** Methode zur Herstellung eines hybriden Proteins nach einem der Ansprüche 1 bis 12, wobei die Methode das Exprimieren eines Fusionsgens umfaßt.

**15.** Hybrides Protein, welches ein Fragment des Diphtherietoxinmoleküls umfaßt, das dem hybriden Protein die Fähigkeit verleiht, die zytoplasmatische Membran einer Zelle zu durchdringen, unter der Voraussetzung, daß das Fragment keinen enzymatisch aktiven Teil des Diphtherietoxin-Fragments A enthält, oder ein Segment des Diphtherietoxins, das an Zellen binden kann, wobei das Fragment kovalent an einen Teil des Polypeptidliganden gebunden ist, der bewirkt, daß das hybride Protein selektiv an die Zelle binden kann.

**16.** Hybrides Protein nach Anspruch 15, wobei der Polypeptidligand Alpha- oder Beta-melanocytenstimulierendes Hormon, Interleukin I, Interleukin II, Interleukin III oder Alpha- oder Beta-HCG ist.

**Patentansprüche für folgenden Vertragsstaat : AT**

**1.** Verfahren für die Herstellung eines hybriden Proteins, das durch ein Fusionsgen kodiert wird, wobei das Fusionsgen folgendes umfaßt:

eine erste Sequenz, die ein Fragment des Diphtherietoxinmoleküls kodiert, welches dem hybriden Protein die Fähigkeit verleiht, die zytoplasmatische Membran einer Zelle zu durchdringen, verschmolzen mit

einer zweiten Sequenz, die einen Teil eines Polypeptidliganden kodiert, der bewirkt, daß das hybride Protein selektiv an die Zelle binden kann,

unter der Voraussetzung, daß das Fusionsgen keinen enzymatisch aktiven Teil des Diphtherietoxin-Fragments A kodiert;

wobei das hybride Protein gegebenenfalls nachweisbar markiert ist

und das Verfahren das Exprimieren des verschmolzenen Gens umfaßt.

**2.** Verfahren nach Anspruch 1, wobei die erste Sequenz nicht das gesamte oder nichts von Fragment A des Diphtherietoxinmoleküls kodiert.

**3.** Verfahren nach Anspruch 1 oder Anspruch 2, wobei die erste Sequenz einen Teil des Fragments B von

12

Diphtherietoxin, der ausreicht, um dem hybriden Protein die Fähigkeit zu verleihen, die zytoplasmatische Membran der Zelle zu durchdringen, und nicht die allgemeine Bindungsregion von Fragment B kodier.t

4. Verfahren nach Anspruch 1 oder Anspruch 3, wobei die erste Sequenz die Sau3A1-Nru1-Sequenz oder die Sau3A1-Sph1-Sequenz des $tox^{228}$-Gens umfaßt.

5. Verfahren nach Anspruch 1 oder Anspruch 3, wobei die erste Sequenz jene Sequenz beinhaltet, welche die amphipatische Region und die hydrophobe Region des Diphtherietoxinmoleküls kodiert.

6. Verfahren nach Anspruch 5, wobei die erste Sequenz ferner eine Sequenz umfaßt, welche die Disulfid-Schleife $l_2$ kodiert.

7. Verfahren nach Anspruch 1, wobei die erste Sequenz die gesamte amphipatische Region und zumindest einen Teil der hydrophoben Region von Fragment B von Diphtherietoxin kodiert, und wobei die erste Sequenz zumindest den Teil der kodierenden Region von Fragment B beinhaltet, der von der Sau3A1-Stelle in der $l_1$-kodierenden Region bis zu einem Punkt reicht, der nicht mehr als 180 Basenpaare von jener Position aufwärts liegt, die der Nru1-Stelle auf dem $tox^{228}$-Allel entspricht.

8. Verfahren nach einem der Ansprüche 1 bis 7, wobei das hybride Protein von der Zelle durch Endocytose aufgenommen werden kann.

9. Verfahren nach Anspruch 8, wobei der Marker ein Fluoreszenzfarbstoff ist.

10. Verfahren nach Anspruch 9, wobei der Farbstoff gemeinsam mit dem hybriden Protein in die zytoplasmatische Membran der Zelle eingefügt werden kann.

11. Verfahren nach einem der vorangehenden Ansprüche, wobei der Polypeptidligand ein Hormon ist.

12. Verfahren nach Anspruch 11, wobei das Hormon Alpha- oder Betamelanocytenstimulierendes Hormon, Interleukin I, Interleukin II, Interleukin III oder Alpha- oder Beta-HCG ist.

13. Methode zur Markierung einer Klasse von Zellen, wobei die Zellen mit einem markierten hybriden Protein in Kontakt gebracht werden, das durch ein Verfahren nach einem der Ansprüche 1 bis 12 hergestellt werden kann.

14. Verfahren zur Herstellung eines hybriden Proteins, das ein Fragment des Diphtherietoxinmoleküls umfaßt, welches dem hybriden Protein die Fähigkeit verleiht, die zytoplasmatische Membran einer Zelle zu durchdringen, unter der Voraussetzung, daß das Fragment keinen enzymatisch aktiven Teil des Diphtherietoxin-Fragments A enthält, oder ein Segment des Diphtherietons, das an Zellen binden kann, wobei das Fragment kovalent an einen Teil des Polypeptidliganden gebunden ist, der bewirkt, daß das hybride Protein selektiv an die Zelle binden kann, und wobei das Verfahren das Exprimieren der DNA umfaßt, die für das hybride Protein kodiert.

15. Verfahren nach Anspruch 14, wobei der Polypeptidligand Alpha- oder Beta-melanocytenstimutierendes Hormon, Interleukin I, Interleukin II, Interleukin III oder Alpha- oder Beta-HCG ist.

FIG 1

FIG 2

FIG 5

14

FIG 3

FIG 4

FIG 6

FIG 7

FIG 8

FIG 9

FIG 10